# EUROPEAN PATENT APPLICATION

(11) **EP 2 466 329 A1**
(43) Date of publication of application: **20.06.2012**
(21) Application number: 11163627.0
(22) Date of filing: 26.04.2011
(51) Int. Cl.: G01S 7/52, A61B 8/08

(54) **Ultrasound system for intuitively displaying motion and method of operating the ultrasound system**

(30) Priority: 17.12.2010 KR 20100129637
(71) Applicant: Samsung Medison Co., Ltd., Nam-myun Hongchun-gun Kangwon do 250-875 (KR)
(72) Inventor: Lee, Jin Yong, Seoul 138-780 (KR); Lee, Bong Heon, Gyeonggi-do 461-190 (KR)
(74) Representative: Schmid, Wolfgang

(57) **Abstract**

Provided is a three-dimensional (3D) ultrasound system (101) for intuitively displaying motion and a method of operating the 3D ultrasound system. The 3D ultrasound system may include a scan unit (103) to generate ultrasonic data of an object through scanning, a coordinate determination unit (105) to determine first coordinates corresponding to a top end of the object, and second coordinates corresponding to a bottom end of the object, from the ultrasonic data, and a display control unit (107) to measure a vector length from the first coordinates to the second coordinates, and to display a color corresponding to the measured vector length by mapping the color to the ultrasonic data.

## Description

### CROSS-REFERENCE TO RELATED APPLICATION

This application claims the benefit of Korean Patent Application No. 10-2010-0129637, filed on December 17, 2010, in the Korean Intellectual Property Office, the disclosure of which is incorporated herein by reference.

### BACKGROUND

### 1. Field of the Invention

The present invention relates to a three-dimensional (3D) ultrasound system and a method of operating the 3D ultrasound system, which may display a color corresponding to a change in motion of an object, thereby enabling intuitive identification of the change in motion.

### 2. Description of the Related Art

An ultrasound system is an apparatus for transmitting, from the surface of a body, an ultrasonic wave signal toward a predetermined structure, that is, an object such as a fetus or an internal organ, inside the body, and for obtaining an image with respect to a cross section of soft tissues or a blood flow using information of the ultrasonic wave signal reflected from the tissues of the body.

This ultrasound system has advantages of a small size, a low cost, a real-time display, and a high stability without exposing patients and users to X-ray radiation and the like, and thus, the ultrasound system is widely used along with other diagnostic imaging systems such as X-ray diagnosis equipment, a computerized tomography (CT) scanner, a magnetic resonance imaging (MRI) equipment, a nuclear medicine diagnosis equipment, and the like.

The ultrasound system may scan the predetermined structure, for example, a left ventricle, and may provide ultrasonic data obtained through the scanning. In this instance, the left ventricle may repeat contraction and relaxation. However, since the ultrasound system may provide only the ultrasonic data with respect to the left ventricle in a state of the contraction, or the left ventricle in a state of the relaxation, it may be difficult for a user to recognize a change in motion with respect to the contraction or the relaxation of the left ventricle based on the ultrasonic data.

### SUMMARY

An aspect of the present invention aims to enable intuitive identification of a change in motion of an object by generating ultrasonic data of the object through scanning, and by displaying a color corresponding to the change in motion of the object by mapping the color to the ultrasonic data.

According to an aspect of the present invention, there is provided a three-dimensional (3D) ultrasound system, including a scan unit to generate ultrasonic data of an object through scanning, a coordinate determination unit to determine first coordinates corresponding to a top end of the object, and second coordinates corresponding to a bottom end of the object, from the ultrasonic data, and a display control unit to measure a vector length from the first coordinates to the second coordinates, and to display a color corresponding to the measured vector length by mapping the color to the ultrasonic data.

The display control unit may map a different brightness of the color, based on proximity to the first coordinates or to the second coordinates.

When the object corresponds to a left ventricle, the coordinate determination unit may determine an apical of the left ventricle to be the first coordinates, and a basal of the left ventricle to be the second coordinates. In this instance, the display control unit may display the color by mapping the color to an area corresponding to wall motion of the left ventricle, among the ultrasonic data.

The display control unit may map a first color of a reddish color when the vector length exceeds the standard and is measured to be increasing, and may map a second color of a bluish color when the vector length is below the standard and is measured to be decreasing.

The 3D ultrasound system may further include a color display bar to guide a color to be mapped based on the vector length.

When the object corresponds to the left ventricle, the color display bar may guide a first color of a reddish color as the color to be mapped when the left ventricle relaxes, and may guide a second color of a bluish color as the color to be mapped when the left ventricles contracts.

When the second coordinates are determined to be plural, the display control unit may display a color corresponding to the vector length measured in association with each of the plurality of the second coordinates, by independently mapping the color.

According to an aspect of the present invention, there is provided a method of operating a 3D ultrasound system, including generating ultrasonic data of an object through scanning, determining first coordinates corresponding to a top end of the object, and second coordinates corresponding to a bottom end of the object, from the ultrasonic data, and measuring a vector length from the first coordinates to the second coordinates, and displaying a color corresponding to the measured vector length by mapping the color to the ultrasonic data.

### EFFECT OF THE INVENTION

According to an aspect of the present invention, it is possible to intuitively identify a change in motion of an object by generating ultrasonic data of the object through scanning, and by displaying a color corresponding to the change in motion of the object by mapping the color to the ultrasonic data.

### BRIEF DESCRIPTION OF THE DRAWINGS

These and/or other aspects, features, and advantages of the invention will become apparent and more readily appreciated from the following description of exemplary embodiments, taken in conjunction with the accompanying drawings of which:
FIG. 1 is a diagram illustrating a structure of a three-dimensional (3D) ultrasound system according to an embodiment of the present invention;
FIG. 2 is a diagram illustrating an example of displaying a color corresponding to changes in motion of an object by mapping the color in a 3D ultrasound system according to an embodiment of the present invention; and
FIG. 3 is a flowchart illustrating a method of operating a 3D ultrasound system according to an embodiment of the present invention.

### DETAILED DESCRIPTION

Reference will now be made in detail to exemplary embodiments of the present invention, examples of which are illustrated in the accompanying drawings, wherein like reference numerals refer to the like elements throughout. Exemplary embodiments are described below to explain the present invention by referring to the figures.

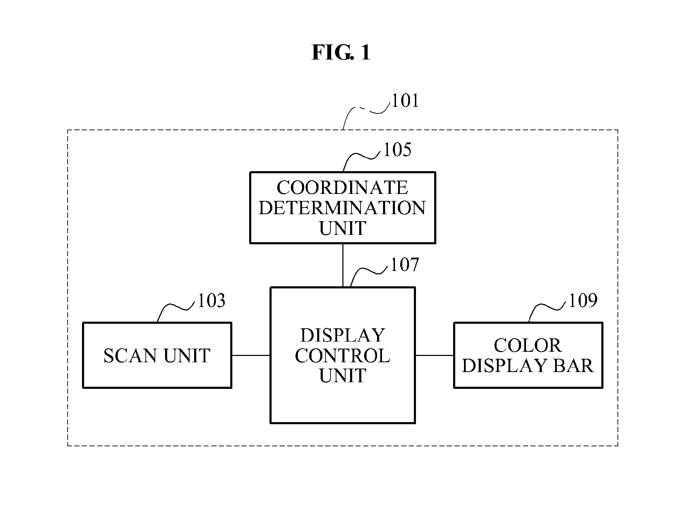

FIG. 1 is a diagram illustrating a structure of a three-dimensional (3D) ultrasound system 101 according to an embodiment of the present invention.

Referring to FIG. 1, the 3D ultrasound system 101 may include a scan unit 103, a coordinate determination unit 105, a display control unit 107, and a color display bar 109.

The scan unit 103 may generate ultrasonic data of an object through scanning. For example, the scan unit 103 may generate the ultrasonic data including image data of a scanned object inside a body. The object inside the body may include an internal organ, for example, a left ventricle. The scan unit 103 may generate the ultrasonic data by scanning the left ventricle.

The coordinate determination unit 105 may determine first coordinates corresponding to a top end of the object, and second coordinates corresponding to a bottom end of the object, from the ultrasonic data.

When the object corresponds to the left ventricle, the coordinate determination unit 105 may determine an apical of the left ventricle to be the first coordinates, and may determine a basal of the left ventricle to be the second coordinates. In this instance, the display control unit 107 may display a color by mapping the color to an area corresponding to wall motion of the left ventricle, among the ultrasonic data, thereby enabling intuitive identification of a change in motion of the wall motion.

Also, the coordinate determination unit 105 may determine a plurality of the second coordinates, by determining left coordinates corresponding to a left end of the bottom end of the object, and right coordinates corresponding to a right end of the bottom end of the object.

The display control unit 107 may measure a vector length from the first coordinates to the second coordinates, and may display a color corresponding to the measured vector length by mapping the color to the ultrasonic data obtained by the scan unit 103. For example, the display control unit 107 may display the color on the ultrasonic data by mapping a first color of a reddish color when the vector length exceeds the standard and is measured to be increasing, and by mapping a second color of a bluish color when the vector length is below the standard and is measured to be decreasing.

The display control unit 107 may display the color by mapping a different brightness of the color based on proximity to the first coordinates or to the second coordinates. That is, the display control unit 107 may set a gray map with respect to the color corresponding to the measured vector length. Then, the display control unit 107 may map a color having a relatively low brightness as being proximate to the first coordinates, and may map a color having a relatively high brightness as being proximate to the second coordinates, based on the gray map. Also, the display control unit 107 may map a color having a relatively high brightness as being proximate to the first coordinates, and may map a color having a relatively low brightness as being proximate to the second coordinates, based on the gray map.

For example, when the object corresponds to the left ventricle, the display control unit 107 may display the color by mapping a different brightness of the color as being proximate to the apical of the left ventricle, or may display the color by mapping a different brightness of the color as being proximate to the basal of the left ventricle.

When the second coordinates are determined to be plural, the display control unit 107 may display the color corresponding to the vector length measured in association with each of the plurality of the second coordinates, by independently mapping the color. That is, when the left coordinates corresponding to the left end of the bottom end of the object, and the right coordinates corresponding to the right end of the bottom end of the object are determined to be the second coordinates, the display control unit 107 may measure a length of a left vector from the first coordinates to the left coordinates, and a length of a right vector from the first coordinates to the right coordinates, and may display colors corresponding to each of the measured lengths of the left vector and the right vector, by independently mapping the colors.

For example, the display control unit 107 may display a first color of a reddish color on ultrasonic data by mapping the first color when the length of the left vector, which may be associated with the left coordinates, exceeds the standard and is measured to be increasing, and may display a second color of a bluish color on the ultrasonic data by mapping the second color when the length of the right vector, which may be associated with the right coordinates, is below the standard and is measured to be decreasing.

The color display bar 109 may guide a color to be mapped based on the vector length from the first coordinates to the second coordinates. For example, the color display bar 109 may be inserted into a side of the ultrasonic data to be displayed. Also, the color display bar 109 may display the first color of the reddish color on an upper end, and may display the second color of the bluish color on a lower end. Thus, it may be identified that the vector length may be increasing when the color mapped, by the display control unit 107, to the ultrasonic data corresponds to the first color of the reddish color. It may also be identified that the vector length may be decreasing when the color mapped, by the display control unit 107, to the ultrasonic data corresponds to the second color of the bluish color.

When the object corresponds to the left ventricle, the color display bar 109 may guide the first color of the reddish color as the color to be mapped when the left ventricle relaxes, and may guide the second color of the bluish color as the color to be mapped when the left ventricle contracts.

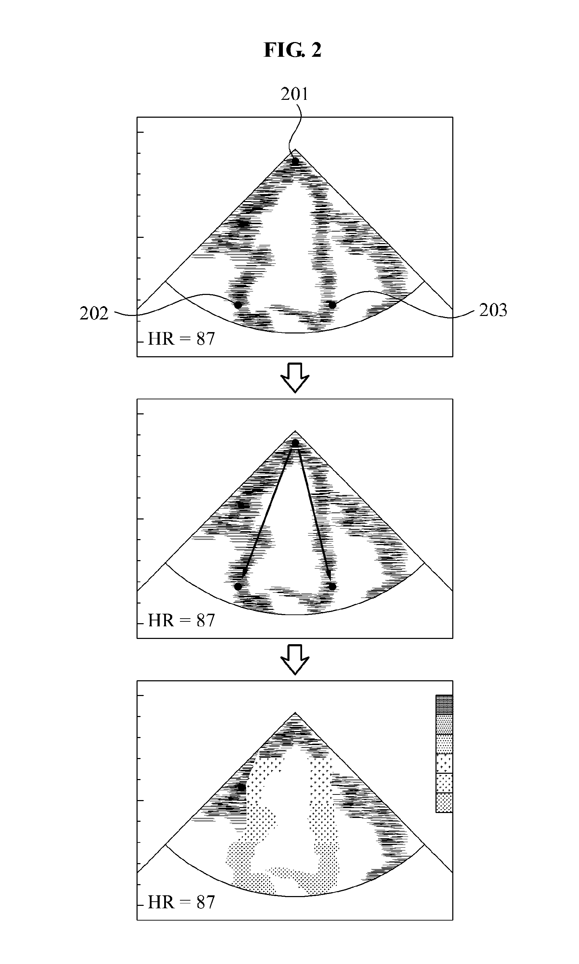

FIG. 2 are diagrams illustrating an example of displaying a color corresponding to a change in motion of an object by mapping the color, in a 3D ultrasound system according to an embodiment of the present invention.

Referring to FIG. 2, the 3D ultrasound system may generate ultrasonic data of a left ventricle through scanning. Also, the 3D ultrasound system may determine an apical of the left ventricle to be first coordinates 201, and may determine left coordinates 202 corresponding to a left end of a basal of the left ventricle, and right coordinates 203 corresponding to a right end of the basal of the left ventricle, among the ultrasonic data.

The 3D ultrasound system may measure a length of a left vector from the first coordinates 201 to the left coordinates 202 and a length of a right vector from the first coordinates 201 to the right coordinates 203, and may display colors corresponding to each of the measured lengths of the left vector and the right vector by independently mapping the colors.

For example, the 3D ultrasound system may display a second color of a bluish color on left wall motion of the ultrasonic data by mapping the second color when the length of the left vector, which may be associated with the left coordinates 202, is below the standard and is measured to be decreasing. Also, the 3D ultrasound system may display the second color of the bluish color on right wall motion of the ultrasonic data by mapping the second color when the length of the right vector, which may be associated with the right coordinates 203, is below the standard and is measured to be decreasing.

In this instance, the 3D ultrasound system may indicate a change in motion on an x-axis with respect to the left wall motion or the right wall motion of the left ventricle, by displaying the color corresponding to the length of the left vector or the length of the right vector by mapping the color. For example, the 3D ultrasound system may indicate that the left wall motion may move to a direction of positive x-axis, that is, right when the second color of the bluish color is displayed by mapping the second color as a color corresponding to the length of the left vector, and may indicate that the right wall motion may move to a direction of negative x-axis, that is, left when the second color of the bluish color is displayed by mapping the second color as a color corresponding to the length of the right vector.

The 3D ultrasound system may display the color display bar 204 on a side of the ultrasonic data, thereby identifying what the color to be mapped to the wall motion of the ultrasonic data may indicate, that is, a change in motion of the left ventricle. For example, the 3D ultrasound system may indicate that the left ventricle may relax when the color to be mapped to the wall motion of the ultrasonic data corresponds to the first color of the reddish color, and may indicate that the left ventricle may contract when the color to be mapped to the wall motion of the ultrasonic data corresponds to the second color of the bluish color, by displaying the color display bar 204 on an upper end of which the first color of the reddish color may be disposed, and on a lower end of which the second color of the bluish color may be disposed.

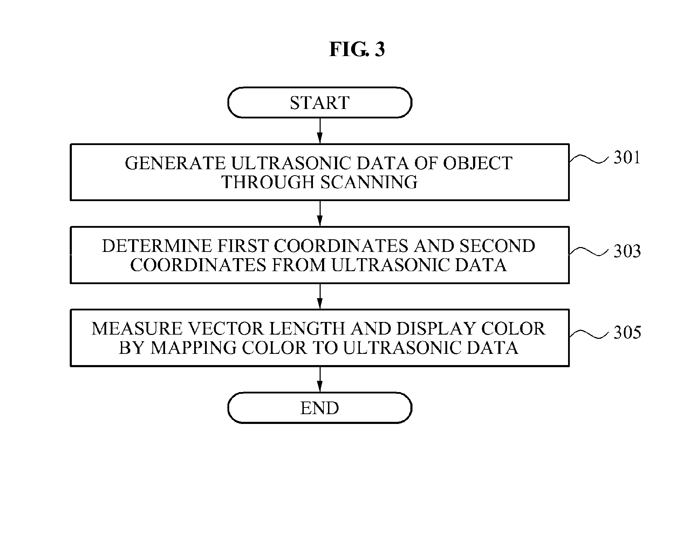

FIG. 3 is a flowchart illustrating a method of operating a 3D ultrasound system according to an embodiment of the present invention.

Referring to FIG. 3, the 3D ultrasound system may generate ultrasonic data of an object through scanning in operation 301. The 3D ultrasound system may obtain image data by scanning a left ventricle as the object, and may generate the ultrasonic data including the obtained image data.

In operation 303, the 3D ultrasound system may determine first coordinates corresponding to a top end of the object, and second coordinates corresponding to a bottom end of the object, from the ultrasonic data. When the object corresponds to the left ventricle, the 3D ultrasound system may determine an apical of the left ventricle to be the first coordinates, and may determine a basal of the left ventricle to be the second coordinates.

Also, the 3D ultrasound system may determine a plurality of the second coordinates, by determining left coordinates corresponding to a left end of the bottom end of the object, and right coordinates corresponding to a right end of the bottom end of the object.

In operation 305, the 3D ultrasound system may measure a vector length from the first coordinates to the second coordinates, and may display a color corresponding to the measured vector length by mapping the color to the ultrasonic data. For example, the 3D ultrasound system may map a first color of a reddish color when the vector length exceeds the standard and is measured to be increasing, and may map a second color of a bluish color when the vector length is below the standard and is measured to be decreasing.

In this instance, when the object corresponds to the left ventricle, the 3D ultrasound system may display a color corresponding to the vector length from the first coordinates to the second coordinates by mapping the color to an area corresponding to wall motion of the left ventricle, among the ultrasonic data.

The 3D ultrasound system may display the color by mapping a different brightness of the color, based on proximity to the first coordinates or the second coordinates. For example, when the object corresponds to the left ventricle, the 3D ultrasound system may display the color by mapping a different brightness of the color as being proximate to the apical of the left ventricle, or may display the color by mapping the different brightness of the color as being proximate to the basal of the left ventricle.

The 3D ultrasound system may display a color display bar, which may guide a color to be mapped based on the vector length, on a side of the ultrasonic data, thereby identifying what the color to be displayed by mapping the color to the ultrasonic data may indicate, that is, a change in motion of the object. When the object corresponds to the left ventricle, the 3D ultrasound system may guide the first color of the reddish color as the color to be mapped when the left ventricle relaxes, and may guide the second color of the bluish color as the color to be mapped when the left ventricle contracts.

When the second coordinates are determined to be plural, the 3D ultrasound system may display the color corresponding to the vector length measured in association with each of the plurality of the second coordinates, by independently mapping the color. For example, when the left coordinates corresponding to the left end of the bottom end of the object, and the right coordinates corresponding to the right end of the bottom end of the object are determined to be the second coordinates, the 3D ultrasound system may measure a length of a left vector from the first coordinates to the left coordinates, and a length of a right vector from the first coordinates to the right coordinates, and may display colors corresponding to each of the measured lengths of the left vector and the right vector, by independently mapping the colors, thereby more accurately indicating the motion of the object.

According to an embodiment of the present invention, it is possible to intuitively identify the change in motion of the object by generating ultrasonic data of the object through scanning, and by displaying a color corresponding to the change in motion of the object by mapping the color to the ultrasonic data.

The above-described exemplary embodiments of the present invention may be recorded in computer-readable media including program instructions to implement various operations embodied by a computer. The media may also include, alone or in combination with the program instructions, data files, data structures, and the like. Examples of computer-readable media include magnetic media such as hard disks, floppy disks, and magnetic tape; optical media such as CD ROM discs and DVDs; magneto-optical media such as floptical discs; and hardware devices that are specially configured to store and perform program instructions, such as read-only memory (ROM), random access memory (RAM), flash memory, and the like. Examples of program instructions include both machine code, such as produced by a compiler, and files containing higher level code that may be executed by the computer using an interpreter. The described hardware devices may be configured to act as one or more software modules in order to perform the operations of the above-described exemplary embodiments of the present invention, or vice versa.

Although a few exemplary embodiments of the present invention have been shown and described, the present invention is not limited to the described exemplary embodiments. Instead, it would be appreciated by those skilled in the art that changes may be made to these exemplary embodiments without departing from the principles and spirit of the invention, the scope of which is defined by the claims and their equivalents.

## Claims

1. An ultrasound system for intuitively displaying a change in motion, the system comprising:
a scan unit to generate ultrasonic data of an object through scanning;
a coordinate determination unit to determine first coordinates corresponding to a top end of the object, and second coordinates corresponding to a bottom end of the object, from the ultrasonic data; and
a display control unit to measure a vector length from the first coordinates to the second coordinates, and to display a color corresponding to the measured vector length by mapping the color to the ultrasonic data.

2. The system of claim 1, wherein the display control unit maps a different brightness of the color, based on proximity to the first coordinates or to the second coordinates.

3. The system of claim 1, wherein when the object corresponds to a left ventricle:
the coordinate determination unit determines an apical of the left ventricle to be the first coordinates, and a basal of the left ventricle to be the second coordinates, and
the display control unit displays the color by mapping the color to an area corresponding to wall motion of the left ventricle, among the ultrasonic data.

4. The system of claim 1, wherein:
the display control unit maps a first color of a reddish color when the vector length exceeds the standard and is measured to be increasing, and
the display control unit maps a second color of a bluish color when the vector length is below the standard and is measured to be decreasing.

5. The system of claim 1, further comprising:
a color display bar to guide a color to be mapped based on the vector length.

6. The system of claim 5, wherein when the object corresponds to a left ventricle:
the color display bar guides a first color of a reddish color as the color to be mapped when the left ventricle relaxes, and
the color display bar guides a second color of a bluish color as the color to be mapped when the left ventricles contracts.

7. The system of claim 1, wherein when the second coordinates are determined to be plural, the display control unit displays a color corresponding to the vector length measured in association with each of the plurality of the second coordinates, by independently mapping the color.

8. A method of operating an ultrasound system, the method comprising:
generating ultrasonic data of an object through scanning;
determining first coordinates corresponding to a top of the object, and second coordinates corresponding to a bottom of the object, from the ultrasonic data; and
measuring a vector length from the first coordinates to the second coordinates, and displaying a color corresponding to the measured vector length by mapping the color to the ultrasonic data.

9. The method of claim 8, wherein the displaying of the color by mapping the color to the ultrasonic data comprises mapping a different brightness of the color based on proximity to the first coordinates or the second coordinates.

10. The method of claim 8, wherein when the object corresponds to a left ventricle:
the determining of the first coordinates and the second coordinates comprises determining an apical of the left ventricle to be the first coordinates, and a basal of the left ventricle to be the second coordinates, and
the displaying of the color by mapping the color to the ultrasonic data comprises displaying the color by mapping the color to an area corresponding to wall motion of the left ventricle, among the ultrasonic data.

11. The method of claim 8, wherein the displaying of the color by mapping the color to the ultrasonic data comprises:
mapping a first color of a reddish color when the vector length exceeds the standard and is measured to be increasing; and
mapping a second color of a bluish color when the vector length is below the standard and is measured to be decreasing.

12. The method of claim 8, further comprising:
displaying a color display bar to guide a color to be mapped based on the vector length.

13. The method of claim 12, wherein when the object corresponds to a left ventricle, the displaying of the color display bar comprises:
guiding a first color of a reddish color as the color to be mapped when the left ventricle relaxes; and
guiding a second color of a bluish color as the color to be mapped when the left ventricles contracts.

14. The method of claim 8, wherein when the second coordinates are determined to be plural, the displaying of the color by mapping the color to the ultrasonic data comprises displaying a color corresponding to the vector length measured in association with each of the plurality of the second coordinates, by independently mapping the color.
